# EUROPEAN PATENT SPECIFICATION

(11) **EP 2 578 176 B1**
(45) Date of publication and mention of the grant of the patent: **14.06.2017**
(21) Application number: 12197042.0
(22) Date of filing: 03.03.2008
(51) Int. Cl.: A61B 18/00

(54) **Variable depth skin heating with lasers**
Durch Laser erfolgende Hauterwärmung von variabler Tiefe
Chauffage de la peau à profondeur variable à l'aide de lasers

(30) Priority: 02.03.2007 US 904598 P
(43) Date of publication of application: 10.04.2013
(62) Divisional of application: 08731281.5
(73) Proprietor: Candela Corporation, Wayland, MA 01778 (US)
(72) Inventor: Dilip, Paithankar Y, Natick, MA 01760 (US); Jones, Christopher J., Leicester, MA 01524 (US); Hsia, James C, Weston, MA 02493 (US)
(74) Representative: Lawrence, John

(56) References cited:
- EP-A2- 0 763 371
- WO-A1-99/49937
- WO-A1-02/053050
- WO-A2-2004/058352
- WO-A2-2005/122694
- WO-A2-2006/076506
- US-A1- 2005 215 987
- US-B1- 6 605 080

## Description

### FIELD OF THE INVENTION

The invention relates generally to treating biological tissue with electromagnetic radiation. The invention relates particularly to treating biological tissue using electromagnetic radiation having at least one parameter related to a target region within the biological tissue to induce a thermal injury within the target region.

### BACKGROUND OF THE INVENTION

Biological tissue (e.g., skin) can be treated by heating a target region within the biological tissue a specific depth. For example, a 1,450 nm based device for relatively shallow sub-surface heating can be used to treat acne. See Paithankar et al., "Acne treatment with a 1,450 nm wavelength laser and cryogen spray cooling," Lasers Surg Med. 31, 106-114 (2002). In another example, relatively deeper sub-surface heating can be used to treat lax skin. See Taub et al., "Multicenter clinical perspectives on a broadband infrared light device for skin tightening," J Drugs Dermatol. 5, 771-778 (2006).

WO 02/053050 discloses performing therapeutic skin treatment by concentrating applied radiation of a selected wavelength at a plurality of treatment portions within a non-treatment portion, including controlling the depth to which radiation is concentrated.

### BRIEF SUMMARY OF THE INVENTION

The invention, as defined by the appended claims, relates to an apparatus for treating by non-invasively delivering electromagnetic radiation to a target region of a biological tissue. The biological tissue can be fatty or adipose tissue, or tissue associated with fat or cellulite. The biological tissue can be skin (e.g., human or mammalian skin), which can exhibit at least one of excess or undesired fat, cellulite, superficial vascular lesion, port wine stain, telangiectasia, small vessel diameter lesion, arterial lesion, capillary lesion, venous lesion, pigmented lesions, pores, scarring, tattoos and other dermatological indications such as acne, oily skin, psoriasis, virtiligo, and the like. The invention can also be used to treat wrinkles, for skin rejuvenation, for hair removal, and for hair regrowth.

In various embodiments, it can be advantageous to deliver electromagnetic radiation through a surface of biological tissue to a target region to induce within the target region a sub-surface thermal injury characterized by a desired degree and a desired depth. It can also be advantageous to confine the sub-surface thermal injury to substantially within the target region. Some embodiments, such as treating cellulite and subcutaneous fat, can require heating target regions at different, specific depths. Furthermore, significant variation can exist in skin thickness because of variation between different anatomical locations and between different patients. Thus, methods and devices that facilitate heating at specific depths can be advantageous. Such embodiments can limit therapeutic injury to a target, spare surrounding tissue, improve treatment efficacy, decrease recovery time, and decrease or eliminate undesirable side effects.

Also disclosed herein is a method for treating biological tissue. The method includes selecting a pulse duration for electromagnetic radiation based on at least one parameter of a target region within the biological tissue. The method also includes delivering the electromagnetic radiation through a surface of the biological tissue to the target region to induce within the target region a sub-surface thermal injury characterized by a desired degree and a desired depth and confining the sub-surface thermal injury to substantially within the target region.

Also disclosed herein is a method for treating a lipid-rich region located at a depth within a target region of a biological tissue. The method includes selecting at least one parameter for electromagnetic radiation based upon the depth of the lipid-rich region and cooling an epidermal region proximal to the target region. The method also includes delivering the electromagnetic radiation through a surface region of the biological tissue to the target region to induce a thermal injury within the lipid-rich region and avoiding substantial undesired thermal injury to the surface region.

Also disclosed herein is a method for treating lipid-rich regions of varying depths within a target region of biological tissue. The method includes selecting at least one first parameter for electromagnetic radiation based upon a first lipid-rich region depth and at least one second parameter for electromagnetic radiation based upon a second lipid-rich region depth. The method also includes delivering electromagnetic radiation having the at least one first parameter through the surface to the target region to induce a thermal injury within the first lipid-rich region and delivering electromagnetic radiation having the at least one second parameter through the surface to the target region to induce a thermal injury within the second lipid-rich region and avoiding substantial undesired surface thermal injury.

The invention includes an apparatus for treating a target region of a biological tissue. The apparatus includes a source of electromagnetic radiation and a controller for selecting at least one parameter of the electromagnetic radiation based upon at least one of a depth and thickness of the target region. The apparatus also includes a device for delivering the electromagnetic radiation through a surface of the biological tissue to the target region, the electromagnetic radiation inducing within the target region a sub-surface thermal injury characterized by a desired degree and a desired depth,

Also disclosed herein is a kit for improving the cosmetic appearance of a subcutaneous fat region in a target region of skin. The kit includes a source of electromagnetic radiation and instruction means. The instruction means include instructions for selecting a pulse duration for electromagnetic radiation based on at least one parameter of a target region within the biological tissue, delivering the electromagnetic radiation through a surface of the biological tissue to the target region to induce within the target region a sub-surface thermal injury characterized by a desired degree and a desired depth, and confining the sub-surface thermal injury to substantially within the target region.

Also disclosed herein is a kit for improving the cosmetic appearance of a subcutaneous fat region in a target region of skin. The kit includes a source of electromagnetic radiation and instruction means. The instruction means include instruction for selecting at least one parameter for electromagnetic radiation based upon the depth of the lipid-rich region, cooling an epidermal region proximal to the target region, delivering the electromagnetic radiation through a surface region of the biological tissue to the target region to induce a thermal injury within the lipid-rich region, and avoiding substantial undesired thermal injury to the surface region.

Also disclosed herein is a kit for improving the cosmetic appearance of a subcutaneous fat region in a target region of skin. The kit includes a source of electromagnetic radiation and instruction means. The instruction means include instructions for selecting at least one first parameter for electromagnetic radiation based upon a first lipid-rich region depth and at least one second parameter for electromagnetic radiation based upon a second lipid-rich region depth, delivering electromagnetic radiation having the at least one first parameter through the surface to the target region to induce a thermal injury within the first lipid-rich region, delivering electromagnetic radiation having the at least one second parameter through the surface to the target region to induce a thermal injury within the second lipid-rich region, and avoiding substantial undesired surface thermal injury.

In other examples, any of the aspects above, or any method, apparatus, or kit described herein, can includes one or more of the following features.

In some aspects methods and/or kits include selecting a wavelength for the electromagnetic radiation based upon the at least one parameter of a target region. Methods and/or kits can include electromagnetic radiation characterized by a wavelength of about 1200 nm or about 1700 nm.

In certain aspects, methods and/or kits include determining at least one parameter of the target region. A parameter can include one or more of cooling timing, cooling temperature, cooling duration, pulse duration, fluence, power density, and wavelength. Methods and/or kits can include determining the depth. The depth can be predetermined or approximated.

In various aspects, methods and/or kits include avoiding substantial undesired thermal injury to the surface of the biological tissue adjacent the target region. Methods and/or kits can include cooling at least a portion of the surface to mitigate substantial undesired surface thermal injury. Methods and/or kits can include avoiding substantial undesired thermal injury to biological tissue underlying the lipid-rich region.

In some aspects, methods and/or kits include determining at least one of the first lipid-rich region depth and the second lipid-rich region depth. Methods and/or kits can include determining at least one of the first lipid-rich region depth and the second lipid-rich region depth using an ultrasonic device. At least one of the first lipid-rich region depth and the second lipid-rich region depth can be predetermined or approximated.

In certain embodiments, the first lipid-rich region and the second lipid-rich region are positioned in different anatomical regions of a single body. The first lipid-rich region and the second lipid-rich region can be positioned in different regions of a single body part. The first lipid-rich region and the second lipid-rich region can be positioned in different regions of a single abdomen.

In various aspects, methods and/or kits include translating an electromagnetic delivery device from a first surface position proximate to the first lipid-rich region to a second surface position proximate to the second lipid-rich and switching the electromagnetic delivery device from the first parameter to the second parameter.

In some embodiments, an apparatus includes a sensor for determining at least one of a depth and thickness of the target region.

In certain embodiments, an apparatus includes a cooling system for cooling at least a portion of the surface to mitigate substantial undesired surface thermal injury.

Other aspects and advantages of the invention will become apparent from the following drawings and description, all of which illustrate principles of the invention, by way of example only.

### BRIEF DESCRIPTION OF THE DRAWINGS

The advantages of the invention described above, together with further advantages, may be better understood by referring to the following description taken in conjunction with the accompanying drawings. The drawings are not necessarily to scale, emphasis instead generally being placed upon illustrating the principles of the invention.
FIG. **1** shows an exemplary cross-section of biological tissue.
FIG. **2** shows an exemplary system for treating skin.
FIG. **3** shows a plot of calculates temperature versus depth for 1 s irradiation.
FIG. **4** shows a plot of calculates temperature versus depth for 4 s irradiation.
FIG. **5** shows a thermal injury resulting from 1,270 nm radiation, stained with NBTC and eosin.
FIG. **6** shows a thermal injury resulting from 1,310 nm radiation, stained with H&E.
FIG. **7** shows a thermal injury resulting from 1,572 nm radiation, stained with H&E.
FIG. **8** shows a thermal injury resulting from 1,450 nm radiation, stained with H&E.
FIG. **9** shows a thermal injury resulting from 1,310 nm, 1 s radiation.
FIG. **10** shows a thermal injury resulting from 1,310 nm, 2 s radiation.
FIG. **11** shows a thermal injury resulting from 1,310 nm, 3 s radiation, stained with H&E.
FIG. **12** shows a thermal injury resulting from 1,130 nm, 3 s radiation, stained with NBTC.
FIG. **13** shows an exemplary network system including a local module and a remote module in communication through a communication network.

### DETAILED DESCRIPTION OF THE INVENTION

The invention is as disclosed in the appended set of claims.
A target region within biological tissue (e.g., skin) can be heated using variable-depth, sub-surface targeting of electromagnetic radiation to induce a therapeutic thermal injury by selection of pulse duration and/or wavelength of the electromagnetic radiation. A treatment depth can be selected using a single device. Such a device can be used to tailor treatments for patients with unique skin thickness, different anatomical locations, or target specific target regions within skin appendages that reside at a known, predetermined, or determinable depth.

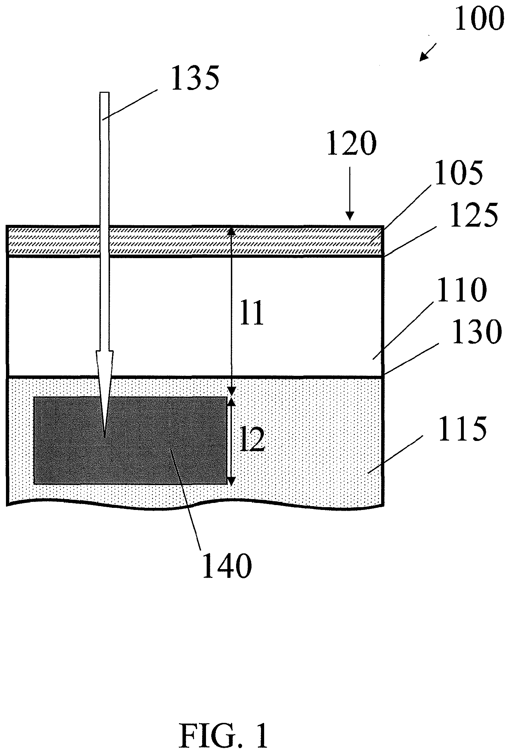

FIG. **1** shows an exemplary cross-section of skin **100** including a region of epidermis **105,** a region of dermis **110,** a region of subcutaneous tissue **115** (e.g., hypodermis), a surface of the skin **120,** and epidermis-dermal interface **125,** and a dermal-subcutaneous interface **130.** In one embodiment, the skin **100** can be a region of human skin. Fatty tissue targeted for treatment can include one or more of fatty tissue within the dermis, subcutaneous fat, cellulite, reserve fat, and intra-cavity fat (not shown).

Electromagnetic radiation **135** can be delivered through the surface of the skin **120** to a target region **140** within the skin (e.g., subcutaneous fatty tissue), to induce within the target region a sub-surface thermal injury characterized by a desired degree and a desired depth. In various embodiments, a target region **140** can include a part or all or any one or all of the epidermis **105,** a region of dermis **110,** a region of subcutaneous fatty tissue **115,** a surface of the skin **120,** an epidermis-dermal interface **125,** and a dermal-subcutaneous interface **130.** A target region **140** can also be in an area of cellulite, reserve fat, or intra-cavity fat. The target region **140** has a depth **I1,** which can be measured as the distance from the surface of the skin **120** to the beginning of the target region **140.** The target region **140** also has a thickness **12.** In various embodiments, a depth **I1** and/or a thickness **12** can be measured relative to any other position or portion of the target region **140.** A target region **140,** and a thermal injury therein, can be confined to an area corresponding to anything from a relatively small anatomical region or feature (e.g., on the order of mm² or a single lesion) to a relatively large anatomical region (e.g., on the order of tens or hundreds of cm² or an abdomen or buttocks). Furthermore, a thermal injury can be continuous, discontinuous, patterned, and/or repeating within the target region **140.** A thermal injury can be confined to substantially within the target region **140.**

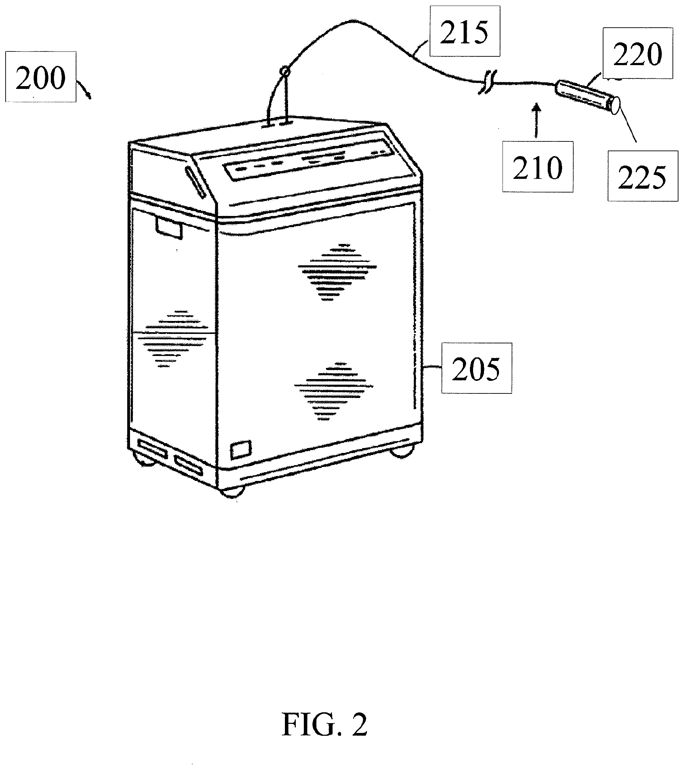

FIG. **2** shows an exemplary embodiment of a system **200 for** treating biological tissue. The system **200** can be used to non-invasively deliver electromagnetic radiation to a target region of biological tissue. The system **200** includes a main unit **205** and a delivery system **210.** In one embodiment, the main unit **205** includes an electromagnetic radiation source that provides electromagnetic radiation directed via the delivery system **210** to a target area. In the illustrated embodiment, the delivery system **210** includes a fiber **215** having a circular cross-section and a handpiece **220.** Electromagnetic radiation can be delivered by the fiber **215** to the handpiece **220,** which can include an optical system (e.g., an optic or system of optics) to direct the beam of radiation to the target area. A user can hold or manipulate the handpiece **220** to irradiate the target area. The handpiece **220** can be positioned in contact with a biological tissue surface, can be positioned adjacent a biological tissue surface, can be positioned proximate a biological tissue surface, can be positioned spaced from a biological tissue surface, or a combination of the aforementioned. In the embodiment shown, the handpiece **220** includes a spacer **225** to space the delivery system **210** from the biological tissue surface. In one embodiment, the spacer **225** can be a distance gauge, which can aid a practitioner with placement of the handpiece **220.** In various embodiments, the system **200** can be an apparatus for treating biological tissue with the electromagnetic radiation.

Sources of electromagnetic radiation can include coherent light sources (e.g., lasers) and incoherent light source (e.g., lamps, light emitting diodes, fluorescent, fluorescent pulsed light, and intense pulse light sources). A light source can be pulsed, continuous, or gated. In one embodiment, a light source can be coupled to a rigid waveguide or a flexible optical fiber or light guide, which can be introduced proximally to a target region of biological tissue.

In some embodiments, a system **200** can include a cooling system that can modulate the temperature in a region of biological tissue and/or minimize unwanted thermal injury to untargeted biological tissue by cooling before, during or after delivery of radiation, or a combination of the aforementioned. A cooling sysmem can also be separate from a system **200.** The delivery system **200** shown in FIG. **2** can cool the biological tissue before, during, or after delivery of radiation, or a combination of the aforementioned. Cooling can include contact conduction cooling, evaporative spray cooling, convective air flow cooling, or a combination of the aforementioned. In one embodiment, the handpiece **220** includes a biological tissue contacting portion that can contact a region of biological tissue. The biological tissue contacting portion can include a sapphire or glass window and a fluid passage containing a cooling fluid. The cooling fluid can be a fluorocarbon type cooling fluid, which can be transparent to the radiation used. The cooling fluid can circulate through the fluid passage and past the window to cool the biological tissue.

A spray cooling device can use cryogen, water, or air as a coolant. In one embodiment, a dynamic cooling device (e.g., a DCD available from Candela Corporation) can cool the biological tissue. For example, the delivery system **200** shown in FIG. **2** can include tubing for delivering a cooling fluid to the handpiece **220.** The tubing can be connected to a container of a low boiling point fluid, and the handpiece can include a valve for delivering a spurt of the fluid to the biological tissue. Heat can be extracted from the biological tissue by evaporative cooling of the low boiling point fluid. In one embodiment, the fluid is a non-toxic substance with high vapor pressure at normal body temperature, such as a Freon or tetrafluoroethane.

In some embodiments, a system **200** or a handpiece **220** can include a device for measure the depth or position of the target region. A device can be an ultrasound. For example, a high frequency ultrasound device can be used. The ultrasound device can deliver ultrasonic energy to measure position of the target region, so that radiation can be directed to the region.

In various embodiments, a system **200** for treating biological tissue and/or an apparatus for treating biological tissue can send and/or receive information to and from a remote site through a network. For example, treatment parameters can be stored remotely and accessed when a particular treatment is selected by a user. This permits an outside source to change, update, or add treatment parameters as new parameters are determined, e.g., by academic research or clinical studies, or by remote practitioners, technicians, operators, or services.

A therapeutic thermal injury can be induced with the electromagnetic radiation in the visible to infrared spectral region. A wavelength that penetrates into at least a portion of biological tissue can be used. A chromophore can include blood (e.g., oxy or deoxy hemoglobin), collagen, melanin, feomelanin, fatty tissue, water, porphryns, and exogenous pigments. The light source can operate at a wavelength with depth of penetration into biological tissue that is less than the thickness of the target region of biological tissue.

In various embodiments, the beam of radiation can have a wavelength between about 400 nm and about 2,600 nm, although longer and shorter wavelengths can be used depending on the application. In some embodiments, the wavelength can be between about 1,000 nm and about 2,200 nm. In other embodiments, the wavelength can be between about 1,160 nm and about 1,800 nm. In yet other embodiments, the wavelength can be between about 1,190 nm and about 1,230 nm or between about 1,700 nm and about 1,760 nm. In one embodiment, the wavelength is about 1,210 nm or about 1,720 nm. In one detailed embodiment, the wavelength is about 1,208 nm, 1,270 nm, 1,310 nm, 1,450 nm, 1,550 nm, 1,720 nm, 1,930 nm, or 2,100 nm. One or more of the wavelengths used can be within a range of wavelengths that can be transmitted to fatty tissue and absorbed by the fatty tissue in the target region of skin.

In various embodiments, the electromagnetic radiation can have a fluence between about 0.1 J/cm² and about 600 J/cm², although higher and lower fluences can be used depending on the application. The electromagnetic radiation can be characterized by an energy density between about 0.1 J/cm² and about 500 J/cm² or between about 10 J/cm² and about 150 J/cm². In one embodiment, the fluence is between about 5 J/cm² and about 100 J/cm². In various embodiments, the electromagnetic radiation delivered to the biological tissue can be characterized by an energy density between about 1 and about 100 60 J/cm², about 2.5 J/cm² and about 60 J/cm², or about 2.5 J/cm² and about 12 J/cm². In certain embodiments, the energy density can be about 1, 5, 10, 50, 100, 150, 200, 250, 300, 350, 400, or 450 J/cm². A system can include a device for selecting a fluence.

In various embodiments, the beam of radiation can have a spot size between about 0.25 mm and about 25 mm, although larger and smaller spot sizes can be used depending on the application. In some embodiments, the electromagnetic radiation can be characterized by a spot size between about 1 mm and about 20 mm. In certain embodiments, a spot size can be up to about 1, 2, 3, 4, or 5 mm in diameter. A system can include a device for selecting a spot size.

In various embodiments, the beam of radiation can have a pulse duration between about 10 µs and about 30 s, although larger and smaller pulse durations can be used depending on the application. A pulse duration can be up to about 100 s. In one embodiment, the beam of radiation can have a pulse duration between about 0.1 s and about 20 s. In one embodiment, the beam of radiation can have a pulse duration between about 1 s and 20 s. In certain embodiments, the beam of radiation can be delivered in a series of sub-pulses spaced in time such that within a region of tissue, the tissue is exposed to radiation intermittently over total time interval of between about 0.1 s and about 20 s. A system can include a device for selecting a pulse duration.

In various embodiments, the beam of radiation can be delivered at a rate of between about 0.01 to about 100 pulses per second or about 0.1 to about 10 pulses per second, although faster, intermediate, or slower pulse rates can be used depending on the application.

In various embodiments, the parameters of the radiation can be selected to deliver the beam of radiation to a predetermined depth. In some embodiments, the radiation can be delivered to the target region up to about 20 mm or up to about 10 mm below an exposed surface of the skin, although shallower or deeper depths can be selected depending on the application. In one embodiment, the radiation is delivered to the target region about 1, 2, 3, 4, 5, 6, 7, 8, 9, 10, 11, 12, 13, 14, 15, 16, 17, 18, 19, or 20 mm below an exposed surface of the skin.

In various embodiments, the tissue can be heated to a temperature of between about 50° C and about 80° C, although higher and lower temperatures can be used depending on the application. In one embodiment, the temperature is between about 50° C and about 70° C. In various embodiments, biological tissue in a target region is heated to a critical temperature to cause thermal injury. In certain embodiments, the critical temperature is below about 100 °C. In other embodiments, the critical temperature is below about 95, 90, 85, 80, 75, 70, 65, 60, 55, or 50 °C. A critical temperature can be a temperature associated with at least one of ablation, coagulation, necrosis, denaturation partial denaturation, and/or acute thermal injury of biological tissue (e.g., various degrees of thermal injury). In addition to temperature, a degree of thermal injury can be affected by a duration of heating

### Exemplary methods

Light-based systems and devices for heating sub-surface target zones at certain or predetermined depths within biological tissue such as skin have numerous therapeutic applications. Selecting one or more parameters for electromagnetic radiation based on at least one parameter of a target region within a biological tissue can affect and control the depth and thickness of a thermal injury. A treatment can include laser irradiation combined with contact cooling. Cooling can facilitate preservation of a top layer of skin (e.g., epidermis, or epidermis and dermis) producing a region of thermally injured tissue under the top layer. The results indicated that the thickness and mean depth of the thermally damaged sub-surface zone can be controlled by choice of laser wavelength and cooling and irradiation times.

One demonstration of the efficacy of such treatments, and a source of data for parameter selection, are Monte Carlo modeling and heat transfer calculations performed to calculate fluence distribution, temperature distribution, and thermal damage for various laser wavelengths in the range of about 1,200-1,800 nm with various pulse durations. Histological evaluation of ex vivo pig skin immediately after various treatments can also demonstrate the efficacy of such treatments, and can be used to empirically determine and select parameters for thermal injury depth and thickness as a function of wavelength and/or pulse duration. Thus, modeled and empirical data can demonstrate that variable depth heating can be achieved through selection of the wavelength and/or laser pulse duration.

At a given wavelength, a variable depth heating device can be constructed by varying cooling and irradiation durations. For example, by choosing appropriate parameters for heating and/or cooling steps, it is possible to selectively heat and thus selectively injure particular zones (e.g., target regions or portions of target regions) within biological tissue such as skin. A target zone can include a chromophore associated with a dermatological condition to be treated or an otherwise endogenous chromophore. By choosing one or more of parameters such as a radiation wavelength, a timing of surface cooling, a cooling temperature, a pulse duration, a radiation fluence and/or a power density (e.g., fluence), a depth, a thickness and/or a degree of thermal injury can be confined to a particular zone within the skin (e.g., one or more of the epidermis, dermis, and subcutaneous tissue). Optimization of the parameters can selectively heat regions of the dermis containing, for example, lipid rich tissue, sebaceous follicles, sebaceous glands, cellulite, vascular targets, hair, or melanin, while substantially or completely sparing overlying regions of epidermis and/or dermis as well as underlying layers of dermis and/or subcutaneous tissue.

Different anatomical sections of the same body can require different depths of treatment. For example, a region of thinner skin (e.g., a face) can require a shallower treatment than a region of thicker skin (e.g., abdomen or leg). Furthermore, different sections of a single region, appendage, or section such as an abdomen, can require multiple depths of treatment. Accordingly, the pulse or cooling or other parameters can be adjusted during a treatment to change a depth of a target region during a treatment. In some embodiments, a pulse duration can be changed while a handpiece is being translated along a body surface to accommodate the depth of a target region. A pulse duration can be longer than a thermal relaxation time of a tissue to induce thermal injury in deeper tissue.

Subcutaneous fat and/or cellulite can be treated by injuring fatty tissue (e.g., a fatty deposit located at or proximate to the dermal interface). A treatment can be accompanied by thickening and/or strengthening of the dermis, which can prevent and/or preclude additional fatty tissue from perturbing the dermal interface. In various embodiments, a treatment can, for example, reduce fat, remove a portion of fat, improve skin laxity, tighten skin, strengthen skin, thicken skin, induce new collagen formation, promote fibrosis of the dermal layer or subcutaneous fat layer, or be used for a combination of the aforementioned.

The treatment radiation can damage one or more fat cells so that at least a portion of lipid contained within can escape or be drained from the treated region. At least a portion of the lipid can be carried away from the tissue through biological processes. In one embodiment, the body's lymphatic system can drain the treated fatty tissue from the treated region. In an embodiment where a fat cell is damaged, the fat cell can be viable after treatment. In one embodiment, the treatment radiation can destroy one or more fat cells. In one embodiment, a first portion of the fat cells is damaged and a second portion is destroyed. In one embodiment, a portion of the fat cells can be removed to selectively change the shape of the body region.

In some embodiments, the beam of radiation can be delivered to the target region to thermally injure, damage, and/or destroy one or more fat cells. For example, the beam of radiation can be delivered to a target chromophore in the target region. Suitable target chromophores include, but are not limited to, a fat cell, lipid contained within a fat cell, fatty tissue, a wall of a fat cell, water in a fat cell, and water in tissue surrounding a fat cell. The energy absorbed by the chromophore can be transferred to the fat cell to damage or destroy the fat cell. For example, thermal energy absorbed by dermal tissue can be transferred to the fatty tissue. In one embodiment, the beam of radiation is delivered to water within or in the vicinity of a fat cell in the target region to thermally injure the fat cell.

In various embodiments, treatment radiation can affect one or more fat cells and can cause sufficient thermal injury in the dermal region of the skin to elicit a healing response to cause the skin to remodel itself. This can result in more youthful looking skin and an improvement in the appearance of cellulite. In one embodiment, sufficient thermal injury induces fibrosis of the dermal layer, fibrosis on a subcutaneous fat region, or fibrosis in or proximate to the dermal interface. In one embodiment, the treatment radiation can partially denature collagen fibers in the target region. Partially denaturing collagen in the dermis can induce and/or accelerate collagen synthesis by fibroblasts. For example, causing selective thermal injury to the dermis can activate fibroblasts, which can deposit increased amounts of extracellular matrix constituents (e.g., collagen and glycosaminoglycans) that can, at least partially, rejuvenate the skin. The thermal injury caused by the radiation can be sufficient to elicit a healing response and cause the fibroblasts to produce new collagen. Excessive denaturation of collagen in the dermis can cause prolonged edema, erythema, and potentially scarring. Inducing collagen formation in the target region can change and/or improve the appearance of the skin of the target region, as well as thicken the skin, tighten the skin, improve skin laxity, and/or reduce discoloration of the skin.

In various embodiments, a zone of thermal injury can be formed at a predetermined location. For example, the peak temperature of the tissue can raised to form a target region at or proximate to the dermal interface. For example, a predetermined wavelength, fluence, pulse duration, and/or cooling parameters can be selected to position the peak of the zone of thermal injury at or proximate to the dermal interface. This can result in collagen being formed at the bottom of the dermis and/or fibrosis at or proximate to the dermal interface. As a result, the dermal interface can be strengthened against fat herniation. For example, strengthening the dermis can result in long-term improvement of the appearance of the skin since new fat being formed or untreated fat proximate the dermal interface can be prevented and/or precluded from crossing the dermal interface into the dermis.

In one embodiment, fatty tissue is heated by absorption of radiation, and heat can be conducted into dermal tissue proximate the fatty tissue. The fatty tissue can be disposed in the dermal tissue and/or can be disposed proximate to the dermal interface. A portion of the dermal tissue (e.g., collagen) can be partially denatured or can suffer another form of thermal injury, and the dermal tissue can be thickened and/or be strengthened as a result of the resulting healing process. In such an embodiment, a fat-selective wavelength of radiation can be used.

In one embodiment, water in the dermal tissue is heated by absorption of radiation. The dermal tissue can have disposed therein fatty tissue and/or can be overlying fatty tissue. A portion of the dermal tissue (e.g., collagen) can be partially denatured or can suffer another form of thermal injury, and the dermal tissue can be thickened and/or be strengthened as a result of the resulting healing process. A portion of the heat can be transferred to the fatty tissue, which can be affected. In one embodiment, water in the fatty tissue absorbs radiation directly and the tissue is affected by heat. In such embodiments, a water selective wavelength of radiation can be used.

A sebaceous follicle disorder can be treated by positioning the target region at a predetermined location. Damage to skin tissue surrounding the sebaceous follicle can be prevented or minimized. In particular, sebaceous follicles and dermal regions containing sebaceous follicles are targeted for thermal injury whereas the underlying dermal and overlaying dermal and epidermal regions are protected from thermal injury. The underlying dermal regions can be protected from thermal injury because, by selection of appropriate parameters, it is possible to limit the penetration depth of the heating energy applied to the region. Accordingly, by choice of appropriate parameters, it is possible to heat skin tissue to a preselected depth thereby sparing the underlying tissue from thermal injury. The overlaying dermal and epidermal regions are protected from thermal injury by appropriate surface cooling. Accordingly, by choice of appropriate heating and cooling parameters, it is possible for the skilled artisan to induce thermal injury to a specific target zone within the dermis of the skin.

For example, in one step, an exposed surface of a preselected region of skin having the disorder (e.g., a lesion characteristic of a sebaceous follicle disorder) is cooled. In a second step, thermal energy is provided in the form of light applied to the preselected region. The thermal energy is applied in an amount and for a time sufficient to induce thermal damage or thermal injury to a portion of the skin containing a sebaceous follicle thereby to reduce or eliminate the production of sebum in the sebaceous follicle or to alter the structure of the sebaceous follicle, for example, by increasing the internal diameter of the follicle, to minimize the possibility of blockage of the follicle, or by decreasing the internal diameter of the follicle. As a result, the treatment can ameliorate one or more skin lesions associated with the sebaceous follicle disorder while at the same time preserving the surface of the skin exposed to the heating energy. The cooling can be one or more of prior to the energy delivery, simultaneous with the energy delivery, and after the energy delivery.

A sebaceous follicle refers to any structure disposed within mammalian, particularly, human, skin, which comprises a hair follicle, also referred to herein as a hair duct, attached to and in fluid flow communication with a sebaceous gland. As a result, sebum produced by the sebaceous gland flows into the hair follicle. The sebaceous follicle optionally may include a hair shaft disposed within the hair follicle. The upper portion of the hair follicle into which sebum is released from the sebaceous gland is referred to as the infundibulum. Sebaceous follicle disorders can result from an over production of sebum by a sebaceous gland of a sebaceous follicle and/or reduction or blockage of sebum flow in the infundibulum of the sebaceous follicle.

Ameliorating the disorder can refer to a decrease in the size of a sebaceous follicle disorder, a decrease in the size of a sebaceous follicle disorder associated lesion, a decrease in density of sebaceous follicle disorder-associated lesions in a preselected region, a decrease or an increase in the size or diameter of a sebaceous follicle or the infundibulum, and/or a decrease in skin-inflammation associated with the sebaceous follicle disorder.

Thermal injury to a sebaceous follicle can result in a structural change and/or a functional change to the sebaceous follicle. For example, sebum over-production can be a factor associated with certain sebaceous follicle disorders. Accordingly, sebaceous gland size and/or sebum production can be reduced in the area afflicted with the disorder. Reduction in sebum production can occur when sebum producing cells disposed within the sebaceous glands are destroyed and thus inactivated, or when their sebum producing activity is reduced. Furthermore, practice of the method of the invention may result in morphological changes to the sebaceous follicle, for example, increasing the diameter of the follicle, to minimize the likelihood of plug formation, or decreasing the diameter of the follicle. By enlarging the size of the follicle, the chance of plug formation is reduced so that any sebum produced by the sebaceous gland can still flow out of the sebaceous follicle. By decreasing the size of the follicle, the size of the disorder or a lesion associated with the disorder can be reduced. The changes can be thermally induced, e.g., by applying light energy, and may result from the temperature-induced cell death and/or protein denaturation or partial denaturation. Accordingly, the temperature of the dermal region containing a sebaceous gland, a sebaceous follicle, and/or the infundibulum can be elevated for a time sufficient to cause cell death and/or protein denaturation or partial denaturation.

In various aspects, a treatment can cause minimal cosmetic disturbance so a patient can return to normal activity following a treatment. For example, a treatment can be performed without causing discernable side effects such as bruising, open wounds, burning, scarring, or swelling. Furthermore, because side effects are minimal, a patient can return to normal activity immediately after a treatment or within a matter of hours, if so desired.

### Monte Carlo, Heat Transfer, and Thermal Damage Calculations

Tissue Optical Properties: Water is a principal absorbtive chromophore in the wavelength range of about 1,100 nm to 2,000 nm in the epidermis and the dermis. Melanin and blood absorption is low in this range. The absorption coefficient of skin is taken as 0.7 times the absorption coefficient of water because skin water content is approximately 70%. The scattering coefficient and the anisotropy coefficient are taken as 100 cm⁻¹ and 0.9, respectively. Approximate (1/e) penetration depth is estimated with the diffusion approximation that takes into account both absorption and scattering. The Monte Carlo (MC) computer program developed by Wang and Jacques is employed for detailed calculations of the fluence distribution. See Wang et al., "MCML - Monte Carlo modeling of photon transport in multi-layered tissues," Computer Methods and Programs in Biomedicine 47:131-146(1995).

In the MC calculations, a 10 mm diameter circular beam was incident on skin, perpendicular to the surface. For the heat transfer calculations, the local fluence multiplied by the absorption coefficient was used as the local volumetric heat source. The heat transfer equation was numerically solved to calculate time dependent temperature field for a set of pre-cooling, laser irradiation, and post-cooling times. The heat transfer coefficient for contact cooling boundary condition was taken as 3,500 W/cm²K. Various pre-cooling, lasing, and post-cooling times were evaluated for the different wavelengths. Typical tissue conduction properties were used. Heat conduction was the only assumed mode of heat transfer. An Arrhenius-type thermal damage integral was evaluated from the time-temperature history at each of the points within skin through the axis through the center of the treatment spot. The parameters for the Arrhenius integral we taken from Pearce et al., "Rate process analysis of thermal damage." Ch. 17 of Welch, ed. "Optical-thermal response of laser-irradiated tissue" New York, Plenum Press (1995) pp 160-162. For each of the calculations, the radiant exposure was adjusted to reach a maximal temperature of 70°C within skin. In a typical non-invasive dermatologic treatment, the peak temperature can be lower.

### Exemplary Ex Vivo Pig Skin Experiments

Fresh pig skin was obtained, stored frozen, and thawed prior to experiments. A contact cooled handpiece with a collimated laser beam was used to treat the skin at various wavelengths and various combinations of pre-laser, post-laser cooling and laser irradiation times. A punch biopsy of the treated skin was taken and stored in 10% buffered formalin solution. Some samples were frozen in OCT. The formalin fixed samples were bisected, sectioned, stained with hematoxylin and eosin (H&E), and observed under an optical microscope for gross thermal damage. The frozen samples were bisected, stained with an nitroblue tetrazolium chloride (NBTC) stain (see Sherwood et al., "Improved staining method for determining the extent of thermal damage to cells," Lasers Surg Med. 2006 Dec 12) and examined under an optical microscope for lack of LDH enzyme activity representative of thermal damage.

### Exemplary results of Monte Carlo and Thermal Calculations

Wavelengths of 1,270 nm, 1,310 nm, 1,572 nm, 1,700 nm, and 1,450 nm were used. The pre-laser cooling and the post-laser cooling times were fixed at 1 s each. Calculations were done for pulse durations of 0.008 s, 0.2 s, 0.5 s, 1.0 s, and 4.0 s. Results confirm that the top layer of the skin is substantially preserved, an intermediate portion experiences thermal injury, and the deep portion of the skin is also substantially preserved. Thus, a sub-surface zone of thermal damage is produced. Exemplary results with 4 s laser irradiation are presented. Higher radiant exposures were needed for wavelengths with lower skin absorption to reach the desired peak maximal temperature of 70°C. Table 2 shows absorption coefficients assumed for the different wavelengths, the (1/e) penetration depths calculated for each wavelength from the diffusion approximation, and depths at which the temperature is maximal during the treatment cycle obtained from the heat transfer calculations. The higher the absorption coefficient, the lower the penetration depth and the lower the depth of maximal temperature and peak thermal damage. The thermal damage zone extends below and above this depth. The zone is narrower for higher absorbing wavelengths and wider for lower absorbing wavelengths. Deep thermal damage, extending past 1.3 mm, can be obtained with less absorbing wavelengths and pulse durations as long as 3 s.

**Table 1. Absorption coefficient, penetration depth, and depth of maximal temperature for various wavelengths.**

| | | | | | |
|---|---|---|---|---|---|
| Wavelength | 1,270 nm | 1,310 nm | 1,450 nm | 1,572 nm | 1,700 nm |
| Absorption coefficient of skin | 0.7 cm⁻¹ | 1.3 cm⁻¹ | 20.5 cm⁻¹ | 5.8 cm⁻¹ | 3.7 cm⁻¹ |
| Penetration Depth | 2.1 mm | 1.5 mm | 0.23 mm | 0.6 mm | 0.8 mm |
| Depth of maximal temperature | 1.15 mm | 1.05 mm | 0.45 mm | 0.75 mm | 0.85 mm |

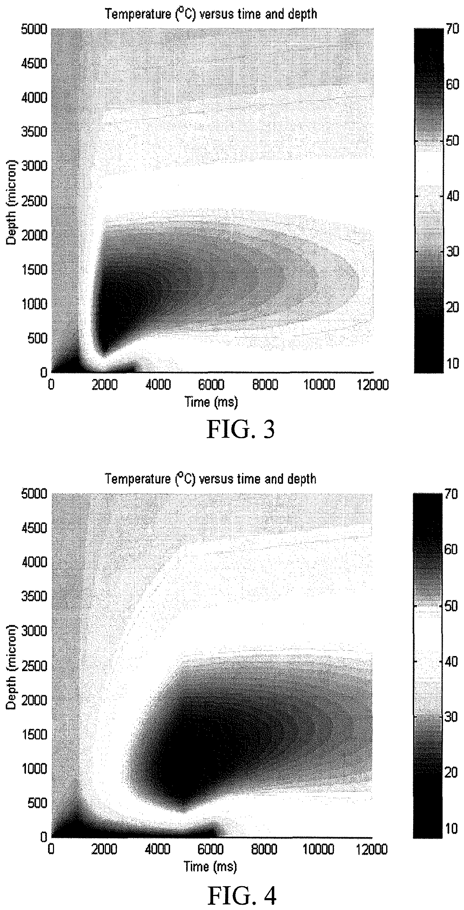

The effect of various pulse durations is at a single wavelength of 1,310 nm are shown in Table 2. The pre-laser cooling time and post-laser cooling times were fixed at 1 s each. Calculations were done for laser irradiation times of 1 s, 2 s, 3 s, and 4 s. With longer pulse durations, higher radiant exposures were needed to reach the desired peak maximal temperature of 70 °C. FIGS. **3** and **4** show plots of temperature versus time and depth through the central axis for pulse durations of 1 s and 4 s, respectively.

The low and high depths were calculated as the depths at which the thermal damage crosses the value of 1. The low and high depths and the mean of the two for the thermally damaged zone are given in Table 2. Also given are the values of radiant exposure for each of the pulse durations.

**Table 2. Mean of low and high depth of thermal damage for various pulse durations.**

| | | | | |
|---|---|---|---|---|
| Pulse Duration | 1 s | 2 s | 3 s | 4 s |
| Mean of High and Low Thermal Damage Depths | 0.96 mm | 1.1 mm | 1.2 mm | 1.3 mm |
| Radiant Exposure used in Calculations | 58 J/cm² | 65 J/cm² | 70 J/cm² | 76 J/cm² |

### Exemplary Ex Vivo Pig Skin Experiments

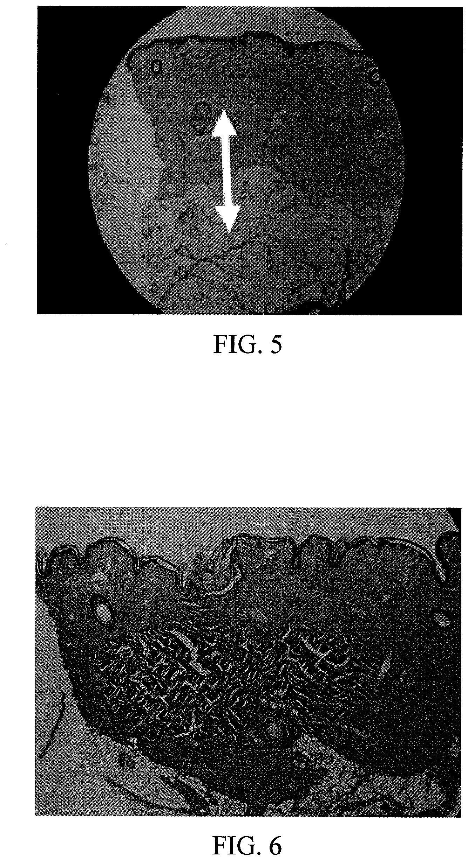

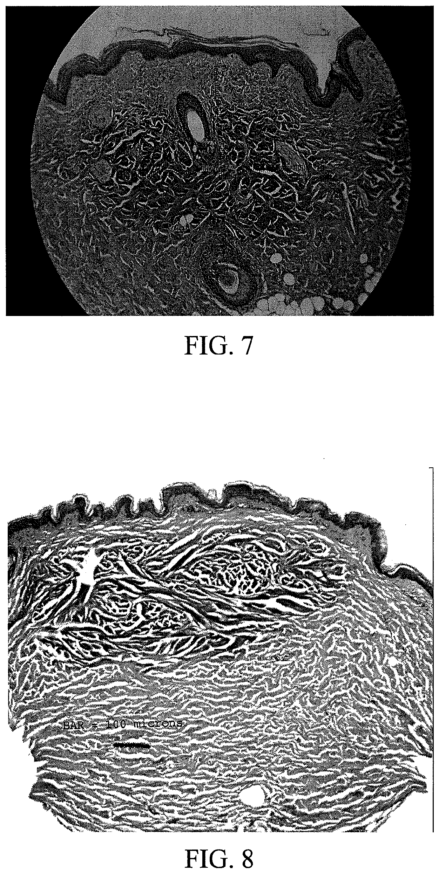

The wavelengths of 1,270 nm, 1,310 nm, 1,450 nm, 1,572 nm, and 1,700 nm were used. For each wavelength, various laser irradiation times were examined. For 1,450 nm and 1,572 nm, the laser irradiation time was 210 ms with 35 ms of DCD setting interspersed within the laser pulse. For the other wavelengths, contact cooling with 3 s laser irradiation was used. The results confirm that the top layer of the skin is preserved, an intermediate portion undergoes thermal damage, and the deep portion of the skin is also preserved. Thus, a sub-surface zone of thermal damage is produced. Images for 1,270 nm, 1,310 nm, and 1,450 nm are presented in FIGS. **5, 6****,** **7,** and **8** respectively. The thermal damage corresponds to a change in color and density. Table 3 shows the mean depth of thermal damage obtained via histological evaluation. With 1,270 nm, damage in subcutaneous fat with its lack of blue staining is noted.

**Table 3. Means of low and high depths of thermal damage for various wavelengths via pig skin histology.**

| | | | | |
|---|---|---|---|---|
| Wavelength, pulse duration | 1,270 nm, | 1,310 nm, | 1,450 nm, | 1,572 nm, |
| | 3 s | 3 s | 0.21 s | 0.21 s |
| Mean of high and low thermal damage depths | 1.5 mm | 1.25 mm | 0.3 mm | 0.6 mm |

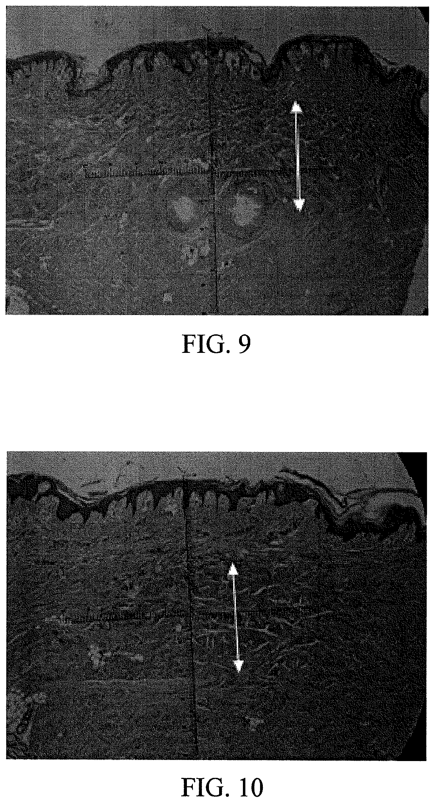

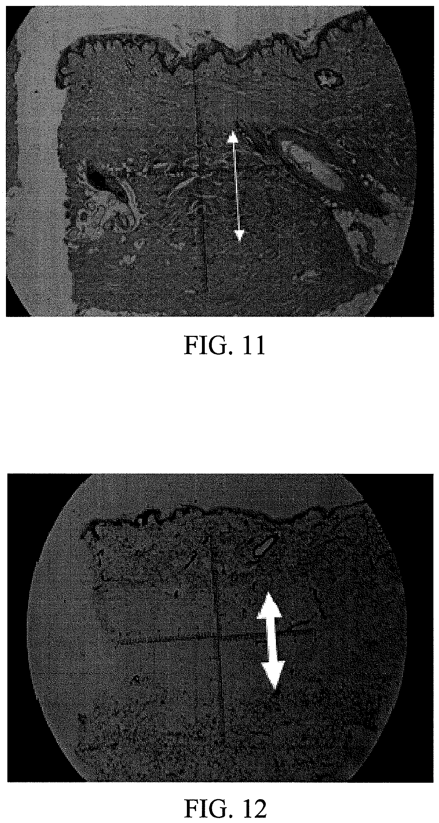

The effect of various pulse durations at a single wavelength of 1,310 nm in pig skin are shown in Table 4. The pre-laser cooling time and post-laser cooling times were 1 s each. Laser irradiation times were 1 s, 2 s, and 3 s. With longer pulse duration and higher radiant exposure was needed to see the desired thermal damage. FIGS. **9, 10**, and **11** show histological slides stained with H&E. The arrows delineate the thermally damaged zones. With longer pulse durations, the thermal injury is deeper. FIG. **12** shows a histological slide stained with NBTC and thermal injury from a 1,310 nm, 3 s laser irradiation. The thermal damage is up to 1.75 mm deep. Table 4 shows the mean depth of thermal damage obtained via histological evaluation.

**Table 4. Means of low and high depths of thermal damage for various pulse durations via pig skin histology.**

| | | | |
|---|---|---|---|
| Pulse Duration, s | 1 | 2 | 3 |
| Mean of High and Low Thermal Damage Depths, mm | 0.875 | 1.0 | 1.25 |
| Example Damage Range, mm | 0.50 - 1.25 | 0.625 - 1.50 | 0.875 - 2.00 |

Paithankar et al. described the use of 1,450 nm radiation and Ramli et al. have described use of 1,064 nm radiation, both combining irradiation with surface cooling to create sub-surface zones of thermal injury in skin. Thermal injury with the highly absorbed 1,450 nm wavelength is relatively shallow while with thermal injury with the less absorbed 1,064 nm wavelength is relatively deep. Intermediate depths of sub-surface thermal injury can be produced. See, Paithankar et al., "Subsurface skin renewal by treatment with a 1450-nm laser in combination with dynamic cooling," J Biomed Opt. 8, 545-51 (2003), Ramli et al., "Subsurface tissue lesions created using an Nd:YAG laser and a sapphire contact cooling probe," Lasers Surg Med 35, 392-396 (2004), and Ramli et al. "Subsurface tissue lesions created using an Nd:YAG laser and cryogen cooling," J Endourol. 17, 923-6 (2003).

The modeled and empirical pig skin results show that one can create sub-surface zones of thermal injury at desired depths via choice of wavelength and pulse duration by combination of laser irradiation and surface cooling. Relatively higher absorbing wavelengths lead to shallower injury while relatively lower absorbing wavelengths lead to deeper injury. Relatively higher absorbing wavelengths lead to thinner (e.g., lesser diameter) injury while relatively lower absorbing wavelengths lead to wider (e.g., greater diameter) injury. Altering pulse duration is another way to control a depth and a width of a sub-surface thermal injury. Longer pulse durations lead to deeper injury while shorter pulse durations lead to shallower injury. Longer pulse durations lead to wider injury while shorter pulse durations lead to thinner injury. Control of depth and/or thickness of thermal injury by selection of parameters including wavelength and/or pulse width provide a powerful tool in developing designer skin treatments that can target specific target regions at specific depths. Such designer or customized treatments can be narrowly tailored to treat a specific condition. Additionally, different depths can be targeted in different treatment sessions or passes in a single session to affect an improvement in skin. Subcutaneous fat can be targeted, for example, since injury with 1,310 nm and 3 s irradiation has been shown to extend as deep as 1.75 mm. Also, it is shown that the modeling based on optical properties of water adequately describes experimental observations in ex vivo pig skin in the wavelength range of 1,200 nm to 1,800 nm.

### Exemplary kits

A kit can be used in a treatment to improve the cosmetic appearance of a region of skin. The kit can include instruction means. The kit can also include a source of electromagnetic radiation. In general, instruction means can include instructions for delivering radiation to a target region. Instructions can include instructions for operating systems, devices, treatments according to the technology. Instructions can also prescribe parameters such as wavelength, power density, pulse duration, and/or cooling parameters for treatment of the target region.

The instruction means (e.g., treatment and/or operation guidelines) can be provided in any form that conveys the requisite information. Instruction means can be audio, for example spoken word, recorded in analog or digital form (e.g., audio recording), or received and/or transmitted in analog or digital form (e.g., by telephone, conference call, or audio signal transmitted over a network). Instruction means can also be visual or video, for example hard-copy (e.g., as a leaflet, booklet, book, manual, recorded medium, and the like) or soft-copy (e.g., recorded in analog or digital form as a file recorded on an optical, electronic, or computer readable medium such as a disk drive, CD-ROM, DVD, and the like). Additionally, instruction means can be interactive or real-time (e.g., a tele-conference or internet chat).

In one example, the kit includes a source of electromagnetic radiation and instruction means. The instruction means include instructions for selecting a pulse duration for electromagnetic radiation based on at least one parameter of a target region within the biological tissue, delivering the electromagnetic radiation through a surface of the biological tissue to the target region to induce within the target region a sub-surface thermal injury characterized by a desired degree and a desired depth, and confining the sub-surface thermal injury to substantially within the target region.

In another example, the kit includes a source of electromagnetic radiation and instruction means. The instruction means include instruction for selecting at least one parameter for electromagnetic radiation based upon the depth of the lipid-rich region, cooling an epidermal region proximal to the target region, delivering the electromagnetic radiation through a surface region of the biological tissue to the target region to induce a thermal injury within the lipid-rich region, and avoiding substantial undesired thermal injury to the surface region.

In still another example, the kit includes a source of electromagnetic radiation and instruction means. The instruction means include instructions for selecting at least one first parameter for electromagnetic radiation based upon a first lipid-rich region depth and at least one second parameter for electromagnetic radiation based upon a second lipid-rich region depth, delivering electromagnetic radiation having the at least one first parameter through the surface to the target region to induce a thermal injury within the first lipid-rich region, delivering electromagnetic radiation having the at least one second parameter through the surface to the target region to induce a thermal injury within the second lipid-rich region, and avoiding substantial undesired surface thermal injury.

In some aspects, the instruction means can be implemented in digital electronic circuitry, or in computer hardware, firmware, software, or in combinations of them. The implementation can be as a computer program product, i.e., a computer program tangibly embodied in an information carrier, e.g., in a machine-readable storage device or in a propagated signal, for execution by, or to control the operation of, data processing apparatus, e.g., a programmable processor, a computer, or multiple computers. A computer program can be written in any form of programming language, including compiled or interpreted languages, and the computer program can be deployed in any form, including as a stand-alone program or as a subroutine, element, or other unit suitable for use in a computing environment. A computer program can be deployed to be executed on one computer or on multiple computers at one site.

The instruction means can be performed by one or more programmable processors executing a computer program to perform functions of the invention by operating on input data and generating output. The instruction means can also be performed by, and an apparatus can be implemented as, special purpose logic circuitry, e.g., an FPGA (field programmable gate array) or an ASIC (application-specific integrated circuit). Subroutines can refer to portions of the computer program and/or the processor/special circuitry that implements that functionality.

Processors suitable for the execution of a computer program include, by way of example, both general and special purpose microprocessors, and any one or more processors of any kind of digital computer. Generally, a processor receives instructions and data from a read-only memory or a random access memory or both. The essential elements of a computer are a processor for executing instructions and one or more memory devices for storing instructions and data. Generally, a computer also includes, or be operatively coupled to receive data from or transfer data to, or both, one or more mass storage devices for storing data, e.g., magnetic, magneto-optical disks, or optical disks. Data transmission and instructions can also occur over a communications network. Information carriers suitable for embodying computer program instructions and data include all forms of non-volatile memory, including by way of example semiconductor memory devices, e.g., EPROM, EEPROM, and flash memory devices; magnetic disks, e.g., internal hard disks or removable disks; magneto-optical disks; and CD-ROM and DVD-ROM disks. The processor and the memory can be supplemented by, or incorporated in special purpose logic circuitry.

To provide for interaction with a user, the above described techniques can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer (e.g., interact with a user interface element). Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input.

The above described techniques can be implemented in a distributed computing system that includes a back-end component, e.g., as a data server, and/or a middleware component, e.g., an application server, and/or a front-end component, e.g., a client computer having a graphical user interface and/or a Web browser through which a user can interact with an example implementation, or any combination of such back-end, middleware, or front-end components. The components of the system can be interconnected by any form or medium of digital data communication, e.g., a communication network. Examples of communication networks include a local area network ("LAN") and a wide area network ("WAN"), e.g., the Internet, and include both wired and wireless networks.

The computing system can include clients and servers. A client and a server are generally remote from each other and typically interact through a communication network. The relationship of client and server arises by virtue of computer programs running on the respective computers and having a client-server relationship to each other.

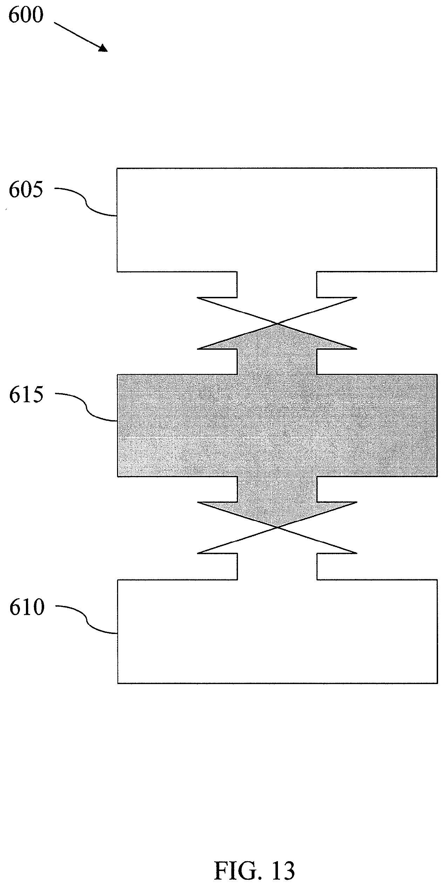

FIG. **13** shows an exemplary network system **600** including a local **605** module and a remote **610** module, which are in communication through a communication network **615.** The local **605** module is configured to provide a treatment, and can include a system **200** for treating biological tissue and/or an apparatus for treating biological tissue. In various embodiments, the local **605** module can include one or more computers, servers, firewalls, databases, or other network devices to process, send, and/or receive information through the communication network **615.** The remote **610** module can include one or more computers, servers, firewalls, databases, or other network devices to process, send, and/or receive information through the communication network **615.** The communication network **615** can be a private company network, for example an intranet, or a public network, for example the internet.

In various embodiments the local **605** module can transmit information to the remote **610** module. For example, the local **605** module can transmit information relating to the biological tissue to be treated and/or information relating to at least one reaction between the biological tissue and the electromagnetic radiation. Based upon the information, the remote **610** module can provide one or more treatment parameters and/or one or more further treatment parameters based upon the information provided. The remote **610** module can calculate treatment parameters and/or retrieve treatment parameters from a database. In some embodiments, the remote **610** module can collect, store, and/or analyze information from multiple treatments by a user and/or multiple users.

While the invention has been particularly shown and described with reference to specific embodiments, it should be understood by those skilled in the art that various changes in form and detail may be made without departing from the scope of the invention as defined by the appended claims.

The invention may encompass one or more of the following aspects:
1. An apparatus for treating a target region of a biological tissue comprising:
   a source of electromagnetic radiation;
   a controller for selecting at least one parameter of the electromagnetic radiation based upon at least one of a depth and thickness of the target region; and
   a device for delivering the electromagnetic radiation through a surface of the biological tissue to the target region, the electromagnetic radiation inducing within the target region a sub-surface thermal injury characterized by a desired degree and a desired depth.
2. The apparatus of aspect 1 wherein the at least one parameter includes a pulse duration.
3. The apparatus of aspect 1 further comprising a sensor for determining at least one of a depth and thickness of the target region.
4. The apparatus of aspect 1 further comprising a cooling system for cooling at least a portion of the surface to mitigate substantial undesired surface thermal injury.
5. The apparatus of aspect 1 wherein the at least one parameter can include one or more of cooling timing, cooling temperature, and cooling duration.
6. A kit for improving the cosmetic appearance of a subcutaneous fat region in a target region of skin, the kit comprising:
   a source of electromagnetic radiation; and
   instruction means including instructions for:
      selecting a pulse duration for electromagnetic radiation based on at least one parameter of a target region within the biological tissue;
      delivering the electromagnetic radiation through a surface of the biological tissue to the target region to induce within the target region a sub-surface thermal injury characterized by a desired degree and a desired depth; and
      confining the sub-surface thermal injury to substantially within the target region.
7. A kit for improving the cosmetic appearance of a subcutaneous fat region in a target region of skin, the kit comprising:
   a source of electromagnetic radiation; and
   instruction means including instructions for:
      selecting at least one parameter for electromagnetic radiation based upon the depth of the lipid-rich region;
      cooling an epidermal region proximal to the target region;
      delivering the electromagnetic radiation through a surface region of the biological tissue to the target region to induce a thermal injury within the lipid-rich region; and
      avoiding substantial undesired thermal injury to the surface region.
8. A kit for improving the cosmetic appearance of a subcutaneous fat region in a target region of skin, the kit comprising:
   a source of electromagnetic radiation; and
   instruction means including instructions for:
      selecting at least one first parameter for electromagnetic radiation based upon a first lipid-rich region depth and at least one second parameter for electromagnetic radiation based upon a second lipid-rich region depth;
      delivering electromagnetic radiation having the at least one first parameter through the surface to the target region to induce a thermal injury within the first lipid-rich region;
      delivering electromagnetic radiation having the at least one second parameter through the surface to the target region to induce a thermal injury within the second lipid-rich region; and
      avoiding substantial undesired surface thermal injury.
9. The kit of aspect 6, 7, and 8 wherein the source includes a fiber coupled laser diode array.
10. The kit of aspect 6, 7, and 8 further comprising a cooling system for cooling an epidermal region of the target region to minimize substantial unwanted injury thereto.
11. The kit of aspect 6, 7, and 8 wherein the instruction means prescribe a wavelength, fluence, and pulse duration for treatment of the subcutaneous fat region.
12. The kit of aspect 6 or 8 wherein the instruction means prescribe cooling at least a portion of the surface of the biological tissue to mitigate substantial undesired surface thermal injury.
13. The kit of aspect 6, 7, and 8 wherein the instruction means prescribe selecting a wavelength for the electromagnetic radiation based upon the at least one parameter of a target region.
14. The kit of aspect 6, 7, and 8 wherein the electromagnetic radiation is characterized by a wavelength of about 1200 nm or about 1700 nm.
15. The kit of aspect 6, 7, and 8 wherein the instruction means prescribe determining at least one parameter of the target region.
16. The kit of aspect 6, 7, and 8 wherein the instruction means prescribe avoiding substantial undesired thermal injury to the surface of the biological tissue adjacent the target region.
17. The kit of aspect 6, 7, and 8 wherein the instruction means prescribe avoiding substantial undesired thermal injury to biological tissue underlying the target region.
18. The kit of aspect 6, 7, and 8 wherein the instruction means prescribe determining the depth.
19. The kit of aspect 6, 7, and 8 wherein the depth is predetermined or approximated.
20. The kit of aspect 6, 7, and 8 wherein the instruction means prescribe at least one parameter can include cooling timing or cooling temperature.
21. The kit of aspect 8 wherein the instruction means prescribe determining at least one of the first lipid-rich region depth and the second lipid-rich region depth.
22. The kit of aspect 8 wherein the instruction means prescribe determining at least one of the first lipid-rich region depth and the second lipid-rich region depth using an ultrasonic device.
23. The kit of aspect 8 wherein at least one of the first lipid-rich region depth and the second lipid-rich region depth is predetermined or approximated.
24. The kit of aspect 8 wherein at least one of the first parameter and the second parameter can include cooling timing, cooling temperature, pulse duration, fluence, and power density.
25. The kit of aspect 8 wherein the first lipid-rich region and the second lipid-rich region are positioned in different anatomical regions of a single body.
26. The kit of aspect 8 wherein the first lipid-rich region and the second lipid-rich region are positioned in different regions of a single body part.
27. The kit of aspect 8 wherein the first lipid-rich region and the second lipid-rich region are positioned in different regions of a single abdomen.
28. The kit of aspect 8 wherein the instruction means prescribe:
   translating an electromagnetic delivery device from a first surface position proximate to the first lipid-rich region to a second surface position proximate to the second lipid-rich; and
   switching the electromagnetic delivery device from the first parameter to the second parameter.

## Claims

1. An apparatus (200) for treating by non-invasively delivering electromagnetic radiation to a target region of a biological tissue, the apparatus comprising:
a main unit (205) including a source of electromagnetic radiation;
a controller for selecting at least one parameter of the electromagnetic radiation based upon at least one of a depth and thickness of the target region;
a delivery system (210) for delivering the electromagnetic radiation through a surface of the biological tissue to the target region, the electromagnetic radiation inducing within the target region a sub-surface thermal injury **characterized by** a desired degree and a desired depth, the delivery system (210) including a fiber (215) having a circular cross-section and a handpiece (220), electromagnetic radiation from the source being delivered by the fiber (215) to the handpiece (220);
a cooling system for cooling at least a portion of the surface to mitigate substantial undesired surface thermal injury; and said apparatus being **characterized in that** it further comprises an ultrasonic sensor for determining the depth and thickness of the target region,
wherein the controller is configured to select a pulse duration of the electromagnetic radiation based upon the depth and thickness of the target region as determined by the ultrasonic sensor and a cooling time and temperature for cooling the portion of the surface to treat the target region by inducing a thermal injury below the surface.

2. The apparatus (200) according to claim 1 wherein the source of electromagnetic radiation is one of a group of sources consisting of a coherent light sources and incoherent light source and wherein the source of electromagnetic radiation is a pulsed, continuous, or gated light source.

3. The apparatus (200) according to claim 2 wherein the source of electromagnetic radiation is coupled to a rigid waveguide or a flexible optical fiber.

4. The apparatus (200) according to any one of claims 2 and 3 wherein the source of electromagnetic radiation generates electromagnetic radiation with wavelengths of 1,000 nm 1,160 nm, 1,190 nm, 1,208 nm, 1,210 nm, 1,230 nm, 1,310 nm, 1,450 nm, 1,550 nm, 1,720 nm, 1,800 nm, 1,930 nm, 2,100 nm and 2,200 nm.

5. The apparatus (200) according any one of claims 2 and 3 wherein the source of electromagnetic radiation generates electromagnetic radiation with fluences of about 0.1 J/cm² and about 600 J/cm² and energy density of about 1, 5, 10, 50, 100, 150, 200, 250, 300, 350, 400, or 450 J/cm²

6. The apparatus according to claim 1 wherein the delivery system (210) includes a handpiece (220) including an optical system to direct the beam of radiation to the target area of the skin and a spacer (225) to space the delivery system (210) from the biological tissue surface.

7. The apparatus according to claim 5 wherein the delivery system (210) includes tubing for delivering a cooling fluid to the handpiece (220), the tubing connected to a container of a low boiling point fluid, and the handpiece further includes a valve for delivering a spurt of the fluid to the biological tissue.

8. The apparatus according to claim 1 wherein the apparatus produces a radiation spot size of about 0.25 mm and about 25 mm and wherein the apparatus includes a device for selecting the spot size.

9. The apparatus according to claim 1 wherein the apparatus produces a beam of radiation with pulse duration between about 10 µs and about 30 sec.

10. The apparatus according to claim 9 wherein beam of radiation is delivered at a rate of between about 0.01 to about 100 pulses per second.

11. The apparatus according to claim 9 wherein the beam of radiation is delivered to the target region of a biological tissue to a depth of about 1, 2, 3, 4, 5, 6, 7, 8, 9, 10, 11, 12, 13, 14, 15, 16, 17, 18, 19, or 20 mm below an exposed surface of the target tissue.

12. The apparatus according to claim 9 wherein the beam of radiation heats the tissue to a temperature of between about 50° C and about 80° C.

## Patentansprüche

1. Vorrichtung (200) zur Behandlung mittels nichtinvasiver Applikation elektromagnetischer Strahlung in einen Zielbereich eines biologischen Gewebes, umfassend:
ein Basisgerät (205) mit einer elektromagnetischen Strahlungsquelle;
ein Steuergerät zur Wahl mindestens eines Parameters der elektromagnetischen Strahlung auf der Grundlage mindestens einer Tiefe und Dicke des Zielbereiches;
ein Applikationssystem (210) zur Applikation der elektromagnetischen Strahlung durch eine Oberfläche des biologischen Gewebes in den Zielbereich, wobei die elektromagnetische Strahlung innerhalb des Zielbereichs eine thermische Verletzung unter der Oberfläche hervorruft, die durch einen gewünschten Grad und eine gewünschte Tiefe gekennzeichnet ist, und wobei das Applikationssystem (210) eine Faser (215) mit kreisförmigem Querschnitt und ein Handstück (220) enthält, wobei elektromagnetische Strahlung von der Quelle über die Faser (215) zum Handstück (220) befördert wird;
ein Kühlsystem zur Kühlung mindestens eines Teils der Oberfläche, um eine wesentliche unerwünschte thermische Verletzung der Oberfläche abzumildern; und
wobei die Vorrichtung dadurch charakterisiert ist, dass sie außerdem einen Ultraschallsensor zur Bestimmung der Tiefe und Dicke des Zielbereichs umfasst;
wobei das Steuergerät so ausgestaltet ist, dass es, zur Behandlung des Zielbereichs durch das Hervorrufen einer thermischen Verletzung unter der Oberfläche, auf der Basis der vom Ultraschallsensor bestimmten Tiefe und Dicke des Zielbereichs eine Impulsdauer der elektromagnetischen Strahlung sowie eine Kühlzeit und -temperatur für das Kühlen des Oberflächenteils wählt.

2. Vorrichtung (200) nach Anspruch 1, wobei die elektromagnetische Strahlungsquelle eine von einer Gruppe von Quellen ist, die aus einer kohärenten Lichtquelle und einer inkohärenten Lichtquelle besteht, und wobei die elektromagnetische Strahlungsquelle eine Impulslicht-, Gleichlicht- oder gattergesteuerte Lichtquelle ist.

3. Vorrichtung (200) nach Anspruch 2, wobei die elektromagnetische Strahlungsquelle mit einem starren Wellenleiter oder einer flexiblen optischen Faser gekoppelt ist.

4. Vorrichtung (200) nach einem der Ansprüche 2 und 3, wobei die elektromagnetische Strahlungsquelle elektromagnetische Strahlung mit den Wellenlängen 1.000 nm, 1.160 nm, 1.190 nm, 1.208 nm, 1.210 nm, 1.230 nm, 1.310 nm, 1.450 nm, 1.550 nm, 1.720 nm, 1.800 nm, 1.930 nm, 2.100 nm und 2.200 nm erzeugt.

5. Vorrichtung (200) nach einem der Ansprüche 2 und 3, wobei die elektromagnetische Strahlungsquelle elektromagnetische Strahlung mit Fluenzen von ca. 0,1 J/cm² und ca. 600 J/cm² und mit einer Energiedichte von ca. 1, 5, 10, 50, 100, 150, 200, 250, 300, 350, 400 oder 450 J/cm² erzeugt.

6. Vorrichtung nach Anspruch 1, wobei das Applikationssystem (210) ein Handstück (220) enthält, das ein optisches System enthält, mit dem das Strahlenbündel auf das Zielgebiet der Haut gerichtet wird, sowie einen Abstandhalter (225), mit dem das Applikationssystem (210) von der Oberfläche des biologischen Gewebes beabstandet wird.

7. Vorrichtung nach Anspruch 5, wobei das Applikationssystem (210) einen Schlauch zur Einleitung einer Kühlflüssigkeit in das Handstück (220) enthält, der Schlauch an einen Behälter mit einer Flüssigkeit von niedrigem Siedepunkt angeschlossen ist und das Handstück außerdem ein Ventil zur Abgabe eines Flüssigkeitsstrahls auf das biologische Gewebe enthält.

8. Vorrichtung nach Anspruch 1, wobei die Vorrichtung Strahldurchmesser von ca. 0,25 mm und ca. 25 mm erzeugt und wobei die Vorrichtung eine Einrichtung zur Wahl des Strahldurchmessers enthält.

9. Vorrichtung nach Anspruch 1, wobei die Vorrichtung einen Strahl mit einer Impulsdauer zwischen ca. 10 µs und ca. 30 s erzeugt.

10. Vorrichtung nach Anspruch 9, wobei ein Strahl mit einer Impulsfrequenz zwischen ca. 0,01 und ca. 100 Impulsen je Sekunde appliziert wird.

11. Vorrichtung nach Anspruch 9, wobei der Strahl auf den Zielbereich eines biologischen Gewebes in einer Tiefe von ca. 1, 2, 3, 4, 5, 6, 7, 8, 9, 10, 11, 12, 13, 14, 15, 16, 17, 18, 19 oder 20 mm unterhalb einer freiliegenden Oberfläche des zu behandelnden Gewebes appliziert wird.

12. Vorrichtung nach Anspruch 9, wobei der Strahl das Gewebe bis auf eine Temperatur zwischen ca. 50°C und ca. 80°C aufheizt.

## Revendications

1. Appareil (200) pour traiter en émettant de manière non invasive un rayonnement électromagnétique sur une région cible d'un tissu biologique, l'appareil comprenant :
une unité principale (205) comprenant une source de rayonnement électromagnétique ;
un dispositif de commande pour sélectionner au moins un paramètre du rayonnement électromagnétique en se basant sur la profondeur et/ou l'épaisseur de la région cible ;
un système d'émission (210) pour émettre le rayonnement électromagnétique à travers une surface du tissu biologique sur la région cible, le rayonnement électromagnétique provoquant dans la région cible une blessure thermique subsuperficielle **caractérisée par** un degré souhaité et une profondeur souhaitée, le système d'émission (210) comprenant une fibre (215) ayant une section transversale circulaire et une pièce à main (220), un rayonnement électromagnétique provenant de la source étant émis par la fibre (215) vers la pièce à main (220) ;
un système de refroidissement pour refroidir au moins une partie de la surface pour atténuer une blessure thermique superficielle importante non désirée ; et
ledit appareil étant **caractérisé en ce qu'**il comprend en outre :
un capteur ultrasonore pour déterminer la profondeur et l'épaisseur de la région cible,
dans lequel le dispositif de commande est configuré pour sélectionner une durée d'impulsion du rayonnement électromagnétique en se basant sur la profondeur et l'épaisseur de la région cible telles que déterminées par le capteur ultrasonore ainsi qu'un temps et une température de refroidissement pour refroidir la partie de la surface afin de traiter la région cible en provoquant une blessure thermique sous la surface.

2. Appareil (200) selon la revendication 1, dans lequel la source de rayonnement électromagnétique est une source d'un groupe de sources composé d'une source de lumière cohérente et d'une source de lumière incohérente et dans lequel la source de rayonnement électromagnétique est une source de lumière pulsée, continue ou à déclenchement périodique.

3. Appareil (200) selon la revendication 2, dans lequel la source de rayonnement électromagnétique est couplée à un guide d'ondes rigide ou une fibre optique flexible.

4. Appareil (200) selon l'une quelconque des revendications 2 et 3, dans lequel la source de rayonnement électromagnétique génère un rayonnement électromagnétique ayant des longueurs d'onde de 1 000 nm, 1 160 nm, 1 190 nm, 1 208 nm, 1 210 nm, 1 230 nm, 1 310 nm, 1 450 nm, 1 550 nm, 1 720 nm, 1 800 nm, 1 930 nm, 2 100 nm et 2 200 nm.

5. Appareil (200) selon l'une quelconque des revendications 2 et 3, dans lequel la source de rayonnement électromagnétique génère un rayonnement électromagnétique ayant des fluences d'environ 0,1 J/cm² et d'environ 600 J/cm² et une densité d'énergie d'environ 1, 5, 10, 50, 100, 150, 200, 250, 300, 350, 400 ou 450 J/cm².

6. Appareil selon la revendication 1, dans lequel le système d'émission (210) comprend une pièce à main (220) comprenant un système optique pour diriger le faisceau de rayonnement vers la zone cible de la peau et une pièce d'écartement (225) pour écarter le système d'émission (210) de la surface de tissu biologique.

7. Appareil selon la revendication 5, dans lequel le système d'émission (210) comprend des tuyaux pour délivrer un fluide de refroidissement à la pièce à main (220), les tuyaux étant raccordés à un récipient d'un fluide à faible point d'ébullition, et la pièce à main comprend en outre une soupape pour délivrer un jet du fluide vers le tissu biologique.

8. Appareil selon la revendication 1, dans lequel l'appareil produit une taille de point de rayonnement d'environ 0,25 mm et d'environ 25 mm et dans lequel l'appareil comprend un dispositif pour sélectionner la taille du point.

9. Appareil selon la revendication 1, dans lequel l'appareil produit un faisceau de rayonnement ayant une durée d'impulsion comprise entre environ 10 µs et environ 30 s.

10. Appareil selon la revendication 9, dans lequel le faisceau de rayonnement est émis à une vitesse comprise entre environ 0,01 et environ 100 impulsions par seconde.

11. Appareil selon la revendication 9, dans lequel le faisceau de rayonnement est émis sur la région cible d'un tissu biologique à une profondeur d'environ 1, 2, 3, 4, 5, 6, 7, 8, 9, 10, 11, 12, 13, 14, 15, 16, 17, 18, 19 ou 20 mm en dessous d'une surface exposée du tissu cible.

12. Appareil selon la revendication 9, dans lequel le faisceau de rayonnement chauffe le tissu à une température comprise entre environ 50 °C et environ 80 °C.
